(19) **Europäisches Patentamt**
**European Patent Office**
**Office européen des brevets**

(11) **EP 4 787 382 A1**

(12) **EUROPEAN PATENT APPLICATION**

(43) Date of publication:
**05.08.2026 Bulletin 2026/32**

(21) Application number: **25219657.1**

(22) Date of filing: **01.12.2025**

(51) International Patent Classification (IPC):
**G16C 20/30** (2019.01)    **G16C 60/00** (2019.01)
**G16C 20/70** (2019.01)

(52) Cooperative Patent Classification (CPC):
**G16C 20/30; G16C 60/00;** G16C 20/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **29.01.2025 JP 2025012869**
**29.07.2025 JP 2025126506**

(71) Applicant: **Sumitomo Riko Company Limited**
**Komaki-shi, Aichi 485-8550 (JP)**

(72) Inventors:
• **KISHIMOTO, Naruhiro**
  **Komaki-shi, Aichi, 485-8550 (JP)**
• **YAJIMA, Takashi**
  **Komaki-shi, Aichi, 485-8550 (JP)**
• **KUMAGAI, Shinji**
  **Komaki-shi, Aichi, 485-8550 (JP)**
• **NAKAICHI, Shingo**
  **Komaki-shi, Aichi, 485-8550 (JP)**
• **KAJITA, Yusuke**
  **Komaki-shi, Aichi, 485-8550 (JP)**

(74) Representative: **Hoffmann Eitle**
**Patent- und Rechtsanwälte PartmbB**
**Arabellastraße 30**
**81925 München (DE)**

(54) **METHOD FOR DESIGNING RUBBER MATERIAL CONTAINING RUBBER COMPONENT AND ADDITIVE**

(57)    A new method for designing a rubber material containing a rubber component and an additive is provided. A method carried out by a computer for designing a rubber material includes:
step S1 of obtaining rubber material data;
step S2 of creating a rubber material dataset;
step S3 of standardizing rubber component and additive blending ratio data and the like;
step S4 of constructing a Gaussian process regression model based on the rubber material dataset;
step S5 of calculating a mean and a standard deviation of a property value predicted in the Gaussian process regression model;
step S6 of calculating an acquisition function;
step S7 of searching for a rubber material based on the acquisition function;
step S8 of reverse-standardizing the rubber component and additive blending ratio data in the rubber material searched for; and
step S9 of obtaining property value data obtained by an experiment of a rubber material produced according to the blending ratio data.

EP 4 787 382 A1

## Description

### TECHNICAL FIELD

The present disclosure relates to a method for designing a rubber material containing a rubber component and an addapparaexplaiitive. More particularly, the present disclosure relates to a computer-aided method for designing a rubber material.

### BACKGROUND ART

In conventional material design, optimization of material composition conditions and the like has been a person-dependent process based on the intuition and experience of skilled workers. However, in recent years, materials informatics has attracted attention to search for optimal materials using information scientific methods that utilize various types of data.

For example, PTL 1 discloses a rubber material property prediction system including: a property prediction processing unit that has a property prediction function created using a model that combines Gaussian process regression with an acquisition function based on rubber material data, and predicts the properties of a rubber material using the property prediction function; and a composition prediction processing unit that predicts composition information of the rubber material using the property prediction function based on the properties of the rubber material.

### RELATED ART DOCUMENT

PTL 1: JP-A-2021-182269

### SUMMARY

### PROBLEMS TO BE SOLVED BY THE DISCLOSURE

In the process of studying methods for designing rubber materials that satisfy the desired property values, the inventors of the present disclosure have conducted intensive research from the viewpoint of improving prediction accuracy, search efficiency, and the like and have succeeded in finding a new design method suitable for rubber materials containing rubber components and additives.

The present disclosure provides a new method for designing a rubber material containing a rubber component and an additive.

Specifically, an exemplary embodiment of the present disclosure provides a design method that may improve the prediction accuracy of a Gaussian process regression model used in a method for designing a rubber material and may improve the efficiency in searching for a desired rubber material.

### MEANS FOR SOLVING THE PROBLEMS

The present disclosure has the following subject matters [1] to [5].

[1] A computer-implemented method for designing a rubber material containing a rubber component and an additive, the method including steps of: obtaining rubber material data including rubber component type data, additive type data, rubber component and additive blending ratio data, and rubber material property value data (S1); creating a rubber material dataset based on the obtained rubber component type data, additive type data, rubber component and additive blending ratio data, and rubber material property value data (S2); standardizing the rubber component and additive blending ratio data and the rubber material property value data in the rubber material dataset (S3); constructing a Gaussian process regression model based on the rubber material dataset including the standardized rubber component and additive blending ratio data and the standardized rubber material property value data (S4); calculating a mean and a standard deviation of a property value predicted in the Gaussian process regression model (S5); calculating an acquisition function based on the mean and the standard deviation of the predicted property value (S6); searching for rubber material data based on the acquisition function (S7); reverse-standardizing the rubber component and additive blending ratio data in the rubber material data searched for (S8); and obtaining property value data obtained by an experiment of a rubber material produced according to the reverse-standardized rubber component and additive blending ratio data (S9).

[2] The method for designing a rubber material containing a rubber component and an additive according to [1], wherein the step (S4) of constructing a Gaussian process regression model is a step of constructing a plurality of Gaussian process regression models based on the rubber material dataset including the standardized rubber component and additive blending ratio data and the standardized rubber material property value data, and the step (S5) of calculating a mean and a standard deviation of a property value is a step of calculating a mean and a standard deviation of a property value predicted in a selected one from the plurality of Gaussian process regression models.

[3] The method for designing a rubber material containing a rubber component and an additive according to [2], wherein the Gaussian process regression models include a first Gaussian process regression model defined by a first kernel function and another Gaussian process regression model defined by a second kernel function, the first kernel function being different from the second kernel function.

[4] The method for designing a rubber material containing a rubber component and an additive according to any one of [1] to [3], wherein the step (S2) of creating a rubber material dataset includes steps of: determining a mode of a blending ratio data group corresponding to any type data included in the rubber material dataset (S2a); and deleting, from the rubber material dataset, rubber material data including blending ratio data other than the mode (S2b).

[5] The method for designing a rubber material containing a rubber component and an additive according to any one of [1] to [4], wherein the acquisition function is a function defined based on a probability that the property value falls within a target range with a predetermined lower limit and a predetermined upper limit.

EFFECTS OF THE DISCLOSURE

According to the present disclosure, a new method for designing a rubber material containing a rubber component and an additive may be provided.

Specifically, according to an exemplary embodiment of the present disclosure, the prediction accuracy of the Gaussian process regression model used in the method for designing a rubber material may be improved, and in addition, the efficiency in searching for a desired rubber material may be improved.

BRIEF DESCRIPTION OF DRAWINGS

FIG. 1 is a flowchart showing an example of a processing procedure of a design method according to an exemplary embodiment of the present disclosure.
FIG. 2 is a chart showing an example of a rubber material dataset according to an exemplary embodiment of the present disclosure.
FIG. 3 is a chart showing an example of a rubber material dataset according to an exemplary embodiment of the present disclosure.

EMBODIMENTS OF THE DISCLOSURE

A method for designing a rubber material containing a rubber component and an additive according to an exemplary embodiment of the present disclosure (which hereinafter may be referred to as "present design method") is carried out by a computer and includes: step S1 of obtaining rubber material data including rubber component type data, additive type data, rubber component and additive blending ratio data, and rubber material property value data; step S2 of creating a rubber material dataset based on the obtained rubber component type data, additive type data, rubber component and additive blending ratio data, and rubber material property value data; step S3 of standardizing the rubber component and additive blending ratio data and the rubber material property value data in the rubber material dataset; step S4 of constructing a Gaussian process regression model based on the rubber material dataset including the standardized rubber component and additive blending ratio data and the standardized rubber material property value data; step S5 of calculating a mean and a standard deviation of a property value predicted in the Gaussian process regression model; step S6 of calculating an acquisition function based on the mean and the standard deviation of the predicted property value; step S7 of searching for rubber material data based on the acquisition function; step S8 of reverse-standardizing the rubber component and additive blending ratio data in the rubber material data searched for; and step S9 of obtaining property value data obtained by an experiment of a rubber material produced according to the reverse-standardized rubber component and additive blending ratio data.

The present design method will be described in detail below.

The present design method relates to a method that performs Gaussian process regression on rubber material data to model the relationship of a property value of a rubber material to the rubber material data, uses the regression result to obtain a property value (mean) of any rubber material data and obtain the variance (standard deviation) of the predicted property value, then calculates an acquisition function based on the mean and the standard deviation, and searches for a condition that maximizes or minimizes the acquisition function.

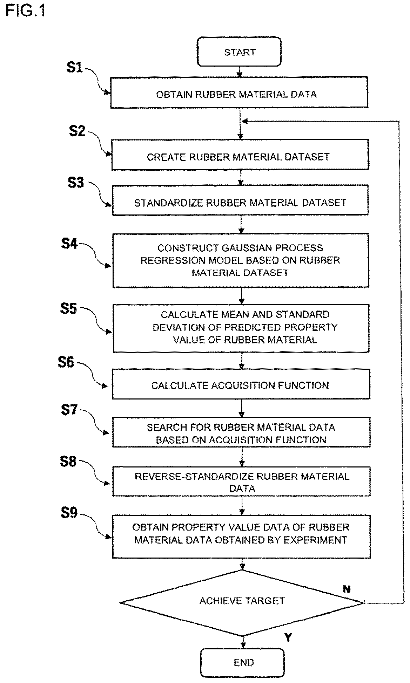

An exemplary embodiment of the present design method is, for example, a design method carried out by a computer to execute processing including each of the steps shown in the flowchart in FIG. 1. The flowchart in FIG. 1 shows an exemplary embodiment, and the present disclosure is not limited to this processing.

The computer that executes the present design method is similar to a computer conventionally used in computer aided engineering (CAE). In the present design method, process routines stored in advance are executed by software and hardware working together in an information processing device such as a personal computer including a central processing unit (CPU), a ROM, a working memory, a storage device such as a magnetic disk, an input device such as a keyboard and a mouse, and a display device such as a display.

<Rubber Material>

The rubber material to be designed in the present design method contains a rubber component and an additive.

Examples of the rubber component include natural rubber, butadiene rubber (BR), isoprene rubber (IR), styrene-butadiene rubber (SBR), liquid isoprene rubber (liquid IR), liquid butadiene rubber (liquid BR), liquid styrene-butadiene rubber (liquid SBR), liquid styrene-isoprene rubber (liquid SI), liquid styrene-ethylene-propylene rubber (liquid SEP), liquid isoprene-butadiene rubber (liquid IR-BR), ethylene-propylene-diene monomer ternary copolymer rubber (EPDM), acrylic rubber, and urethane rubber. These may be used alone or in combination of two or more.

The additive includes various additives added according to a predetermined purpose. Examples of the additive include vulcanizing agents, vulcanization accelerators, vulcanization accelerator aids, vulcanization retardants, anti-aging agents, antioxidants, reinforcing agents, fillers, plasticizers, tackifiers, lubricants, peptizers, coloring agents, and various polymer components (excluding rubber components). These may be used alone or in combination of two or more.

Examples of the rubber material property value include mechanical properties, thermal properties, and electrical and electronic properties. Specific examples include tensile strength, hardness, breaking strength, and elongation at break. The present design method sets a target value(s) for one or two or more property values and designs a rubber material that satisfies the target value(s).

<Steps S1 to S2>

The present design method carried out by a computer includes: step S1 of obtaining rubber material data including rubber component type data, additive type data, rubber component and additive blending ratio data, and rubber material property value data; and step S2 of creating a rubber material dataset based on the rubber component type data, the additive type data, the rubber component and additive blending ratio data, and the rubber material property value data.

Examples of the rubber component and additive type data include information indicating type and grade (brand name). The rubber component and additive type data includes, for example, information indicating molecular structure, information indicating mechanical properties, and the like.

The rubber component and additive blending ratio data is, for example, information indicating the blending amount of rubber component and the blending amount of additive to constitute a rubber material on a mass basis.

The rubber material property value data is information indicating, for example, mechanical property value data, thermal property value data, electrical and electronic property value data, and the like of a rubber material containing a rubber component and an additive.

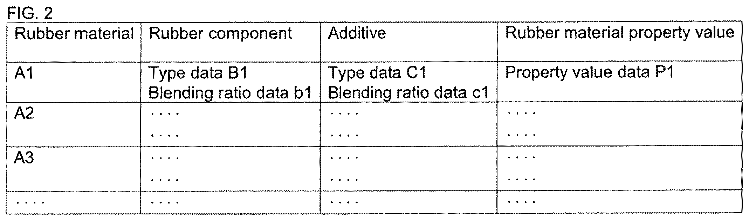

The rubber material dataset includes, for example, rubber component type data, additive type data, rubber component and additive blending ratio data, and rubber material property value data. FIG. 2 shows an example of the rubber material dataset. As shown in FIG. 2, the rubber material dataset includes multiple pieces of rubber material data, such as rubber material data A1, rubber material data A2, and rubber material data A3. Rubber material data A1 at least includes type data B1 indicating the type of rubber component and blending ratio data b1 of the rubber component; type data C1 indicating the type of additive and blending ratio data c1 of the additive; and property value data P1 indicating a property value such as tensile strength of rubber material A1. Rubber material data A1, rubber material data A2, and rubber material data A3 are data related to rubber materials different from each other, and at least one of the respective type data or blending ratio data is different from each other. The rubber material dataset is created based on constraints, for example, the type and the blending ratio of rubber components and additives, according to the purpose of design.

As a specific example of embodiments of the present design method, the present design method is preferably used as a method for designing a rubber material containing a rubber component and two or more additives. In this embodiment, the rubber material data includes the type data and the blending ratio data for each of the two or more additives. The rubber material data may also include other data such as production condition data. Examples of the production condition data include production condition data (temperature, time, order, etc.) in a material kneading process and production condition data (temperature, time, etc.) in a vulcanization process after the kneading process.

As a specific example of embodiments of the present design method, from the viewpoint of improving the prediction accuracy and improving the efficiency in searching for a desired rubber material, it is preferable that step S2 of creating a rubber material dataset includes: step S2a of determining the mode of a blending ratio data group corresponding to any type data included in the rubber material dataset; and step S2b of deleting, from the rubber material dataset, rubber material data including blending ratio data other than the mode.

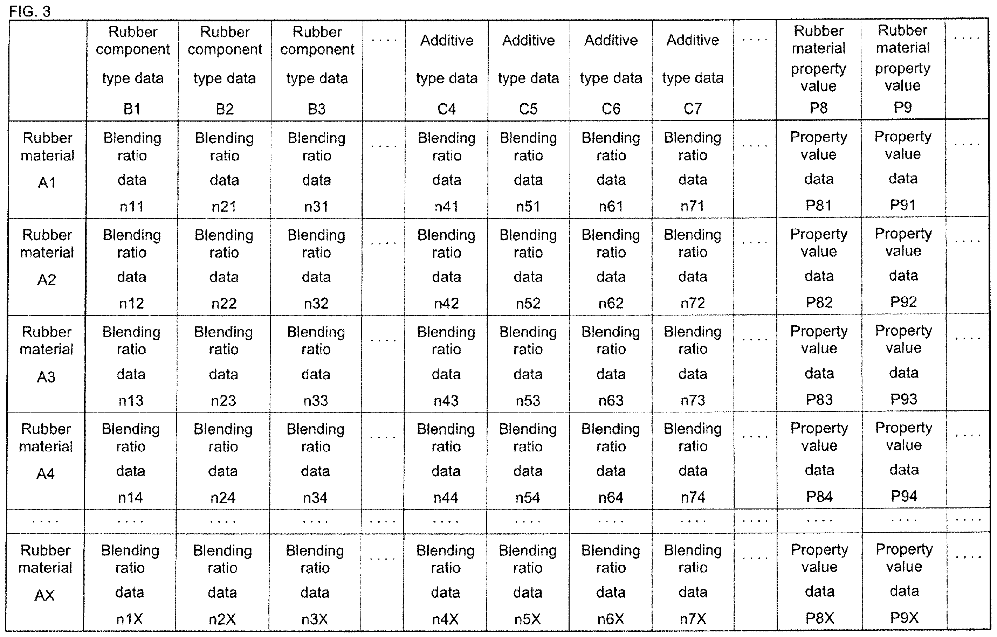

Referring to FIG. 3, step S2a determines the mode of a blending ratio data group (e.g., "blending ratio data n11 to n1X," "blending ratio data n41 to n4X," etc.) corresponding to the type data (e.g., "type data B1," "type data C4," etc.) of any rubber component or any additive included in the rubber material dataset. Using additive type data C4 as an example, data that appears most frequently is determined based on each of the data in the blending ratio data group (n41 to n4X) (e.g., if X=1000, based on 1000 pieces of data). Specifically, the value of each data is identified: for example, blending ratio data n41 is identified as "5(%)," blending ratio data n42 as "5(%)," blending ratio data n43 as "5(%)," blending ratio data n44 as "10(%)," ... (omitted) ..., blending ratio data n4X as "8(%)," and the value that appears most frequently (e.g., "5 (%)") is determined.

The term "mode" in the present disclosure implies both a specific value and a range from a specific value to a specific value (mode range). As an example of the mode range, although not limited to, the entire range with maximum and minimum values of any blending ratio data may be divided into arbitrary ranges, and the range that appears most frequently may be used as the mode range.

Next, step S2b deletes, from the rubber material dataset, rubber material data including blending ratio data other than the mode determined as described above. Specifically, referring to FIG. 3, the mode is determined based on the blending ratio data group (n41 to n4X) corresponding to type data C4 of any additive included in the rubber material dataset, and the rubber material data including blending ratio data different from the mode in the blending ratio data group (n41 to n4X) is deleted. For example, if blending ratio data n41 is "5(%)," blending ratio data n42 is "5(%)," blending ratio data n43 is "5(%)," blending ratio data n44 is "10(%)," ... (omitted) ..., blending ratio data n4X is "8(%)," and the mode is "5(%)," rubber material data A4 (data in a row corresponding to "rubber material data A4" in FIG. 3) including blending ratio data n44 different from the mode is deleted from the rubber material dataset, and rubber material data AX (data in a row corresponding to "rubber material data AX" in FIG. 3) including blending ratio data n4X different from the mode is deleted from the rubber material dataset.

If the mode of the blending ratio data group corresponding to any type data determined in step S2a is "0 (%)," the rubber material data including blending ratio data different from the mode (0 (%)) may be deleted from the rubber material dataset, and this type data may be further deleted from the rubber material dataset. For example, if the mode of the blending ratio data group corresponding to rubber component type data B3 is "0 (%)" and blending ratio data n31 is a value (e.g., "2 (%)") other than the mode (0 (%)), rubber material data A1 (data in a row corresponding to "rubber material data A1" in FIG. 3) may be deleted from the rubber material dataset, and data corresponding to this type data B3 (data in a column corresponding to "type data B3" in FIG. 3) may be further deleted from the rubber material dataset.

As a specific example of embodiments of steps S2a and S2b, if a condition of using one or more specific rubber components and one or more specific additives is given according to the desired design specifications, first, the type data of the corresponding rubber components and the type data of the corresponding additives are selected. For example, referring to FIG. 3, rubber component type data B1, additive type data C6, and additive type data C7 are selected from the rubber material dataset according to the desired design specifications. Then, the above steps S2a and S2b are performed for each of the rubber component type data (type data B2 and B3) and the additive type data (type data C4 and C5) that have not been selected. In other words, the mode is determined for each of the blending ratio data groups corresponding to the non-selected type data B2, B3, C4, and C5, and the rubber material data including blending ratio data other than the mode is deleted from the rubber material dataset. Specifically, taking type data C5, which is the non-selected type data, as an example, each value is identified: blending ratio data n51 is identified as "0(%)," blending ratio data n52 as "1(%)," blending ratio data n53 as "2(%)," blending ratio data n54 as "2(%)," ... (omitted) ..., and blending ratio data n5X as "2(%)." If the mode is "2(%)," rubber material data A1 including blending ratio data n51 which is different from the mode is deleted from the rubber material dataset, and rubber material data A2 including blending ratio data n52 which is different from the mode is deleted from the rubber material dataset. In this way, performing the above processing (steps S2a and S2b) for each of the non-selected type data B2, B3, C4, and C5 may reduce noise otherwise caused by rubber components and additives that are not taken into consideration according to the design specifications, thereby increasing the prediction accuracy of the constructed Gaussian process regression model. In addition, the search efficiency may be improved by dimensionality reduction.

In particular, in designing rubber materials in which a wide variety of raw materials are assumed to be used (e.g., more than 150 types of rubber components and additives in total), it is difficult to accumulate a wide variety of rubber material data, and the total number of pieces of rubber material data tends to be small. However, the design method performing steps S2a and S2b of the present disclosure is useful to improve the prediction accuracy and the efficiency in searching for a desired rubber material even when the total number of pieces of rubber material data is small. The design method performing steps S2a and S2b of the present disclosure is particularly useful as a method for designing a rubber material with high experimental costs because the dimensionality reduction may narrow down the experimental range and improve the search efficiency.

In this technical field, the total number of pieces of rubber material data corresponding to each row shown in FIG. 3 is, for example, about 50 to 1000, or at most about 2000 in many cases. The total number of pieces of type data corresponding to each column shown in FIG. 3 is, for example, about 50 to 200 in many cases. The total number of pieces of type data selected when the condition of using one or more specific rubber components and one or more specific additives is given according to the desired design specifications is about 5 to 30, and often about 8 to 25.

<Step S3>

The present design method includes step S3 of standardizing the rubber component and additive blending ratio data and the rubber material property value data in the rubber material dataset from the viewpoint of improving the prediction accuracy of the Gaussian process regression model or the search efficiency.

Step S3 standardizes, as scaling of the rubber material dataset, at least the rubber component blending ratio data, the additive blending ratio data, and the property value data that constitute the rubber material data. In a specific example of embodiments of step S3, the blending ratio data of each rubber component is converted so that the mean of the rubber component blending ratio data that constitutes the rubber material dataset is 0 and the standard deviation is 1. Similarly, the blending ratio data of each additive is converted so that the mean of the additive blending ratio data that constitutes the rubber material dataset is 0 and the standard deviation is 1. Similarly, each property value data is converted so that the mean of the property value data that constitutes the rubber material dataset is 0 and the standard deviation is 1. Each data after standardization is obtained by subtracting the mean from each data before standardization and dividing the subtraction result by the standard deviation.

Each standardized blending ratio data and each standardized property value data are represented, for example, by formula (1).

$$X'=(X-\mu x)/\sigma x \qquad (1)$$

[In formula (1), X' is the data after standardization, X is the data before standardization, $\mu x$ is the mean of the data, and $\sigma x$ is the standard deviation of the data.]

As a specific example of embodiments of step S3, although not limited to the following, the rubber material dataset is divided into a training dataset and a test dataset, and the blending ratio data of each rubber component is converted so that the mean of the rubber component blending ratio data that constitutes the training dataset is 0 and the standard deviation is 1. Similarly, the blending ratio data of each additive is converted so that the mean of the additive blending ratio data that constitutes the training dataset is 0 and the standard deviation is 1. Similarly, each property value data is converted so that the mean of the rubber material property value data that constitutes the training dataset is 0 and the standard deviation is 1.

The blending ratio data and the property value data that constitute the test dataset may be standardized using, for example, the mean and the standard deviation of the training dataset, although not limited thereto.

<Steps S4 to S7>

The present design method includes: step S4 of constructing a Gaussian process regression model based on the dataset including the standardized rubber component and additive blending ratio data and the standardized rubber material property value data; step S5 of calculating a mean and a standard deviation of a property value predicted in the Gaussian process regression model; step S6 of calculating an acquisition function based on the mean and the standard deviation of the predicted property value; and step S7 of searching for rubber material data based on the acquisition function.

The Gaussian process regression model in the present design method is constructed by machine learning, where the rubber component type data, the additive type data, and the rubber component and additive blending ratio data are explanatory variables, and the rubber material property value data is an objective variable. The Gaussian process regression model is a prediction model that uses the rubber component type data, the additive type data, and the rubber component and additive blending ratio data as inputs, and the predicted property value of a rubber material as an output.

Examples of the kernel function defined in the Gaussian process regression model include Radial Basis Function (RBF), Matern kernel, linear kernel, exponential kernel, and periodic kernel. The kernel function may be a combination of two or more different kernel functions by addition, or multiplication, or addition and multiplication.

In a specific example of embodiments of the present design method, it is preferable to construct a plurality of Gaussian process regression models and select any Gaussian process regression model from the viewpoint of increasing the accuracy of the Gaussian process regression model and efficiently searching for a desired rubber material. More specifically, in a specific example of embodiments of the present disclosure, it is preferable that the Gaussian process regression models include a Gaussian process regression model defined by a kernel function and another Gaussian process regression model defined by a kernel function different from the kernel function.

In a specific example of embodiments of the present disclosure, it is preferable to calculate the accuracy ($R^2$, etc.) of each of the constructed Gaussian process regression models and select any Gaussian process regression model based on the superiority of the accuracy of the Gaussian process regression models.

The Gaussian process regression model may predict the mean and the standard deviation in the modeled objective function. In the present design method, the mean and the standard deviation of the predicted property value of any rubber material data are calculated using the Gaussian process regression model, the acquisition function is calculated based on the mean and the standard deviation of the predicted property value of the rubber material data, and rubber material data that satisfies a predetermined property value (target value) is searched for based on the acquisition function.

Examples of the acquisition function include Upper Confidence Bound (UCB), Lower Confidence Bound (LCB), Probability in Target Range (PTR), Mutual Information (MI), Probability of Improvement (PI), and Expected Improvement (EI).

The number of pieces of rubber material data generated as a result of the search based on the acquisition function is not limited, but may be one or more, and two or more pieces of rubber material data may be searched for.

In designing a rubber material, it is required to simultaneously satisfy multiple properties (usually 4 to 15 properties, or more properties in some cases), including properties that are contradictory to each other (e.g., elongation at break and heat resistance). In this respect, with acquisition functions such as Probability of Improvement (PI), which uses the probability of updating the maximum value in an existing dataset, and Upper Confidence Bound (UCB) and Lower Confidence Bound (LCB), which control the search width by assigning an arbitrary coefficient to PI, extreme formulations that specialize in a particular property value are often developed, so that many experimental trials are necessary to develop a formulation that balances multiple property values required for a rubber material, raising concerns about excessive experimental workload. Therefore, from the viewpoint of efficiently optimizing the multiple property values required for the design of rubber materials, it is preferable to use a function defined based on the probability that the property value falls within a target range with a predetermined lower limit and upper limit (Probability in Target Range (PTR)) as an acquisition function. The use of PTR as an acquisition function allows more efficient optimization of a rubber material that simultaneously satisfies multiple property values than when other acquisition functions are used.

<Steps S8 to S9>

The present design method includes step S8 of reverse-standardizing the rubber component and additive blending ratio data in the rubber material data searched for; and step S9 of obtaining the property value data obtained by an experiment of a rubber material produced according to the reverse-standardized rubber component and additive blending ratio data.

The rubber material data searched for in step S7 includes the standardized rubber component and additive blending ratio data. In step S8, reverse standardization of the rubber component blending ratio data and the additive blending ratio data is performed. Each reverse-standardized blending ratio data is represented, for example, by formula (2).

$$X = X' \cdot \sigma x + \mu x \qquad (2)$$

[In formula (2), X is the data before standardization (reverse-standardized data), X' is the data after standardization, $\mu x$ is the mean of the data, and $\sigma x$ is the standard deviation of the data.]

A rubber material is produced according to the blending ratio data after the reverse standardization. The blending ratio data obtained by reverse standardization is, for example, information indicating the blending amount of rubber component and the blending amount of additive to constitute a rubber material on a mass basis. In the present design method, a rubber material is produced using the blending ratio data. The property value of the produced rubber material is measured using an experimental device. A computer obtains the property value data of the rubber material obtained by measurement using the experimental device.

If the property value of the rubber material measured using the experimental device meets the desired target value, the design of the rubber material may be terminated. For example, if the value of the elongation at break of the rubber material measured using a tensile tester or the like meets a predetermined target value, the design of the rubber material may be terminated.

<Other Steps>

On the other hand, if the property value of the rubber material measured using the experimental device does not meet the desired target value, the search for rubber material data is continued according to the same steps as above.

In other words, an exemplary embodiment of the present design method includes step S10 of adding, to the rubber material dataset, rubber material data including the rubber component type data, the additive type data, and the rubber component and additive blending ratio data in the rubber material data searched for, and the property value data obtained by the experiment. In an exemplary embodiment of the present design method, the above steps are repeated again after step S10 to continue the search for rubber material data.

Specifically, in an exemplary embodiment of the present design method, the following steps are performed: standardizing the rubber component and additive blending ratio data and the rubber material property value data in the rubber material dataset updated by step S10; constructing a Gaussian process regression model based on the rubber material dataset including the standardized rubber component and additive blending ratio data and the standardized rubber material property value data; calculating a mean and a standard deviation of a property value predicted in the Gaussian process regression model; calculating an acquisition function based on the mean and the standard deviation of the predicted property value; searching for rubber material data based on the acquisition function; reverse-standardizing the rubber component and additive blending ratio data in the rubber material data searched for; and obtaining property value data obtained by an experiment of a rubber material produced according to the reverse-standardized rubber component and additive blending ratio data.

If the property value of the rubber material measured using the experimental device meets a desired target value, the design of the rubber material may be terminated. On the other hand, if the desired target value is not met, the above steps are repeated again, and the search for rubber material data is continued until the desired target value is met.

An exemplary embodiment of the present disclosure is extremely useful in that it improves the prediction accuracy of the Gaussian process regression model used in the method for designing a rubber material and also improves the efficiency in searching for a desired rubber material.

<Design Apparatus for Rubber Materials>

An exemplary embodiment of the present disclosure provides a design apparatus for designing a rubber material using software that executes each of the above steps. Specifically, for example, an apparatus for designing a rubber material uses software for causing a computer to execute: step S1 of obtaining rubber material data including rubber component type data, additive type data, rubber component and additive blending ratio data, and rubber material property value data; step S2 of creating a rubber material dataset based on the obtained rubber component type data, additive type data, rubber component and additive blending ratio data, and rubber material property value data; step S3 of standardizing the rubber component and additive blending ratio data and the rubber material property value data in the rubber material dataset; step S4 of constructing a Gaussian process regression model based on the rubber material dataset including the standardized rubber component and additive blending ratio data and the standardized rubber material property value data; step S5 of calculating a mean and a standard deviation of a property value predicted in the Gaussian process regression model; step S6 of calculating an acquisition function based on the mean and the standard deviation of the predicted property value; step S7 of searching for rubber material data based on the acquisition function; step S8 of reverse-standardizing the rubber component and additive blending ratio data in the rubber material data searched for; and step S9 of obtaining property value data obtained by an experiment of a rubber material produced according to the reverse-standardized rubber component and additive blending ratio data.

According to the present disclosure, a new method for designing a rubber material containing a rubber component and an additive may be provided. According to an exemplary embodiment of the present disclosure, the prediction accuracy of the Gaussian process regression model used in the method for designing a rubber material may be improved, and in addition, the efficiency in searching for a desired rubber material may be improved.

**Claims**

1. A computer-implemented method for designing a rubber material comprising a rubber component and an additive, the method comprising steps of:

   obtaining rubber material data including rubber component type data, additive type data, rubber component and additive blending ratio data, and rubber material property value data (S1);
   creating a rubber material dataset based on the obtained rubber component type data, additive type data, rubber component and additive blending ratio data, and rubber material property value data (S2);
   standardizing the rubber component and additive blending ratio data and the rubber material property value data in the rubber material dataset (S3);
   constructing a Gaussian process regression model based on the rubber material dataset including the standardized rubber component and additive blending ratio data and the standardized rubber material property value data (S4);
   calculating a mean and a standard deviation of a property value predicted in the Gaussian process regression model (S5);
   calculating an acquisition function based on the mean and the standard deviation of the predicted property value (S6);
   searching for rubber material data based on the acquisition function (S7);
   reverse-standardizing the rubber component and additive blending ratio data in the rubber material data searched for (S8); and
   obtaining property value data obtained by an experiment of a rubber material produced according to the reverse-standardized rubber component and additive blending ratio data (S9).

2. The method for designing a rubber material comprising a rubber component and an additive according to claim 1, wherein the step (S4) of constructing a Gaussian process regression model is a step of constructing a plurality of Gaussian process regression models based on the rubber material dataset including the standardized rubber component and additive blending ratio data and the standardized rubber material property value data, and the step (S5) of calculating a mean and a standard deviation of a property value is a step of calculating a mean and a standard deviation of a property value predicted in a selected one from the plurality of Gaussian process regression models.

3. The method for designing a rubber material comprising a rubber component and an additive according to claim 2, wherein the Gaussian process regression models include a first Gaussian process regression model defined by a first kernel function and another Gaussian process regression model defined by a second kernel function, the first kernel function being different from the second kernel function.

4. The method for designing a rubber material comprising a rubber component and an additive according to any one of claims 1 to 3, wherein the step (S2) of creating a rubber material dataset includes steps of: determining a mode of a blending ratio data group corresponding to any type data included in the rubber material dataset (S2a); and deleting, from the rubber material dataset, rubber material data including blending ratio data other than the mode (S2b).

5. The method for designing a rubber material comprising a rubber component and an additive according to any one of claims 1 to 4, wherein the acquisition function is a function defined based on a probability that the property value falls within a target range with a predetermined lower limit and a predetermined upper limit.

FIG.1

```
                        ┌──────────────┐
                        │    START     │
                        └──────┬───────┘
                               │
S1 ──┐       ┌─────────────────▼──────────────────┐
     └──────▶│     OBTAIN RUBBER MATERIAL DATA     │
             └─────────────────┬──────────────────┘
                               │
S2 ──┐       ┌─────────────────▼──────────────────┐◀──────┐
     └──────▶│    CREATE RUBBER MATERIAL DATASET   │       │
             └─────────────────┬──────────────────┘       │
                               │                           │
S3 ──┐       ┌─────────────────▼──────────────────┐       │
     └──────▶│  STANDARDIZE RUBBER MATERIAL DATASET│       │
             └─────────────────┬──────────────────┘       │
                               │                           │
S4 ──┐       ┌─────────────────▼──────────────────┐       │
     └──────▶│      CONSTRUCT GAUSSIAN PROCESS     │       │
             │   REGRESSION MODEL BASED ON RUBBER  │       │
             │           MATERIAL DATASET          │       │
             └─────────────────┬──────────────────┘       │
                               │                           │
S5 ──┐       ┌─────────────────▼──────────────────┐       │
     └──────▶│    CALCULATE MEAN AND STANDARD      │       │
             │   DEVIATION OF PREDICTED PROPERTY   │       │
             │        VALUE OF RUBBER MATERIAL     │       │
             └─────────────────┬──────────────────┘       │
                               │                           │
S6 ──┐       ┌─────────────────▼──────────────────┐       │
     └──────▶│    CALCULATE ACQUISITION FUNCTION   │       │
             └─────────────────┬──────────────────┘       │
                               │                           │
S7 ──┐       ┌─────────────────▼──────────────────┐       │
     └──────▶│    SEARCH FOR RUBBER MATERIAL DATA  │       │
             │     BASED ON ACQUISITION FUNCTION   │       │
             └─────────────────┬──────────────────┘       │
                               │                           │
S8 ──┐       ┌─────────────────▼──────────────────┐       │
     └──────▶│   REVERSE-STANDARDIZE RUBBER MATERIAL│      │
             │                DATA                 │       │
             └─────────────────┬──────────────────┘       │
                               │                           │
S9 ──┐       ┌─────────────────▼──────────────────┐       │
     └──────▶│  OBTAIN PROPERTY VALUE DATA OF RUBBER│      │
             │ MATERIAL DATA OBTAINED BY EXPERIMENT│       │
             └─────────────────┬──────────────────┘       │
                               │                           │
                          ┌────▼────┐                 N    │
                         ╱           ╲─────────────────────┘
                        ╱  ACHIEVE     ╲
                        ╲   TARGET     ╱
                         ╲            ╱
                          └────┬─────┘
                               │ Y
                        ┌──────▼───────┐
                        │     END      │
                        └──────────────┘
```

FIG. 2

| Rubber material | Rubber component | Additive | Rubber material property value |
|---|---|---|---|
| A1 | Type data B1<br>Blending ratio data b1 | Type data C1<br>Blending ratio data c1 | Property value data P1 |
| A2 | . . . .<br>. . . . | . . . .<br>. . . . | . . . .<br>. . . . |
| A3 | . . . .<br>. . . . | . . . .<br>. . . . | . . . .<br>. . . . |
| . . . . | . . . . | . . . . | . . . . |

FIG. 3

| | Rubber component type data B1 | Rubber component type data B2 | Rubber component type data B3 | · · · · | Additive type data C4 | Additive type data C5 | Additive type data C6 | Additive type data C7 | · · · · | Rubber material property value P8 | Rubber material property value P9 | · · · · |
|---|---|---|---|---|---|---|---|---|---|---|---|---|
| Rubber material A1 | Blending ratio data n11 | Blending ratio data n21 | Blending ratio data n31 | · · · · | Blending ratio data n41 | Blending ratio data n51 | Blending ratio data n61 | Blending ratio data n71 | · · · · | Property value data P81 | Property value data P91 | · · · · |
| Rubber material A2 | Blending ratio data n12 | Blending ratio data n22 | Blending ratio data n32 | · · · · | Blending ratio data n42 | Blending ratio data n52 | Blending ratio data n62 | Blending ratio data n72 | · · · · | Property value data P82 | Property value data P92 | · · · · |
| Rubber material A3 | Blending ratio data n13 | Blending ratio data n23 | Blending ratio data n33 | · · · · | Blending ratio data n43 | Blending ratio data n53 | Blending ratio data n63 | Blending ratio data n73 | · · · · | Property value data P83 | Property value data P93 | · · · · |
| Rubber material A4 | Blending ratio data n14 | Blending ratio data n24 | Blending ratio data n34 | · · · · | Blending ratio data n44 | Blending ratio data n54 | Blending ratio data n64 | Blending ratio data n74 | · · · · | Property value data P84 | Property value data P94 | · · · · |
| · · · · | · · · · | · · · · | · · · · | · · · · | · · · · | · · · · | · · · · | · · · · | · · · · | · · · · | · · · · | · · · · |
| Rubber material AX | Blending ratio data n1X | Blending ratio data n2X | Blending ratio data n3X | · · · · | Blending ratio data n4X | Blending ratio data n5X | Blending ratio data n6X | Blending ratio data n7X | · · · · | Property value data P8X | Property value data P9X | · · · · |

EP 4 787 382 A1

EUROPEAN SEARCH REPORT

Application Number

EP 25 21 9657

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROMÁN ALLEN JONATHAN ET AL: "Natural Rubber Blend Optimization via Data-Driven Modeling: The Implementation for Reverse Engineering", POLYMERS, vol. 14, no. 11, 31 May 2022 (2022-05-31), page 2262, XP093396658, CH ISSN: 2073-4360, DOI: 10.3390/polym14112262 * pp. 1-2 p. 4 p. 7 p. 9 pp. 23-24; tables 1,2,6 * | 1-5 | INV. G16C20/30 G16C60/00 ADD. G16C20/70 |
| A | Brochu Eric ET AL: "A Tutorial on Bayesian Optimization of Expensive Cost Functions, with Application to Active User Modeling and Hierarchical Reinforcement Learning", arXiv.org, 1 December 2010 (2010-12-01), pages 1-49, XP093396668, Retrieved from the Internet: URL:https://arxiv.org/pdf/1012.2599 * pp. 10-14 * | 1-5 | TECHNICAL FIELDS SEARCHED (IPC) G16C |
| A | US 11 216 737 B2 (UNCOUNTABLE INC [US]) 4 January 2022 (2022-01-04) * the whole document * | 1-5 | |

The present search report has been drawn up for all claims

| Place of search | Date of completion of the search | Examiner |
|---|---|---|
| Munich | 11 May 2026 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 787 382 A1

## ANNEX TO THE EUROPEAN SEARCH REPORT
## ON EUROPEAN PATENT APPLICATION NO.

EP 25 21 9657

11-05-2026

| Patent document cited in search report | Publication date | Patent family member(s) | Publication date |
|---|---|---|---|
| US 11216737 B2 | 04-01-2022 | US 2019057313 A1 | 21-02-2019 |
| | | US 2022114462 A1 | 14-04-2022 |

**EP 4 787 382 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021182269 A